# EUROPEAN PATENT SPECIFICATION

(11) **EP 1 846 401 B1**
(45) Date of publication and mention of the grant of the patent: **18.06.2008**
(21) Application number: 06706532.6
(22) Date of filing: 01.02.2006
(51) Int. Cl.: C07D 455/02, C07D 487/04, A61K 31/4709, A61P 33/06

(54) **NEW 4-AMINOQUINOLINE DERIVATIVES AS ANTIMALARIALS**
NEUE 4-AMINOCHINOLINDERIVATE ALS ANTIMALARIAMITTEL
NOUVEAUX DÉRIVÉS DE 4-AMINOQUINOLÉINE COMME COMPOSÉS ANTIMALARIA

(30) Priority: 04.02.2005 EP 05425051
(43) Date of publication of application: 24.10.2007
(73) Proprietor: CTG Pharma S.r.l., 20131 Milan (IT)
(72) Inventor: SPARATORE, Fabio, I-16131 Genoa (IT)
(74) Representative: Monti, Rinaldo
(86) International application number: PCT/EP2006/000846
(87) International publication number: WO 2006/082030

(56) References cited:
- WO-A-20/05037833
- US-A- 5 596 002
- US-A1- 2004 152 730
- SPARATORE A ET AL: "QUINOLIZIDINE DERIVATIVES WITH ANTIMICROBIAL ACTIVITY DERIVATI CHINOLIZIDINICI AD ATTIVITA ANTIMICROBICA" IL FARMACO, ROME, IT, vol. 42, no. 3, March 1987 (1987-03), pages 159-174, XP009043709 ISSN: 0014-827X
- BOIDO CANU C ET AL: "PREPARAZIONE E ATTIVITA ANTILEUCEMICA DI CHINOLIZIDINIL-ALCHIL DERIVATI DELLA 4-AMMINOCHINOLINA E DELLA 9-AMMINOACRIDINA" BOLLETTINO CHIMICO FARMACEUTICO, SOCIETA EDITORIALE FARMACEUTICA, MILANO, IT, vol. 128, 1989, pages 212-215, XP009043694 ISSN: 0006-6648
- CHAUHAN P M S ET AL: "ANTIPARASITIC AGENTS: PART 5-SYNTHESIS OF 4-(SUBSTITUTED ARYL)AMINO-7-CHLOROQUINOLINES AS POTENTIAL ANTIMALARIAL AGENTS" INDIAN JOURNAL OF CHEMISTRY, JODHPUR, IN, vol. 25B, November 1986 (1986-11), pages 1142-1145, XP009037752
- BAILEY, DENIS M.: "Quinoline antimalarials. Folded chloroquine" J. MED. CHEM., vol. 12, no. 1, 1969, pages 184-185, XP002337636
- TARA SINGH, ROBERT G. STEIN, JOHN F. HOOPS, JOHN H. BIEL, WALLACE K. HOYA, DEANNA R. CRUZ: "Antimalarials. 7-Chloro-4-(substituted amino)quinolines" J. MED. CHEM., vol. 14, no. 4, 1971, pages 283-286, XP002337637
- PAUL A. STOCKS, KAYLENE J. RAYNES, PATRICK G. BRAY, B. KEVIN PARK, PAUL M. O'NEILL, STEPHEN A. WARD: "Novel Short Chain Chloroquine Analogues Retain Activity Against Chloroquine Resistant K1 Plasmodium falciparum" J. MED. CHEM., vol. 45, 2002, pages 4975-4983, XP002337638
- TARA SINGH, ROBERT G. STEIN, HARLAN H. KOELLING, JOHN F. HOOPS, JOHN H. BIEL: "Antimalarials. Some Quinuclidine Derivatives of 7-Chloro-4-aminoquinoline and 6-Methoxy-8-aminoquinoline" J. MED. CHEM., vol. 12, 1969, pages 524-526, XP002337639
- YOSHIAKI TAKAYA, HIDEHISA TASAKA, TOMOYUKI CHIBA, KOJI UWAI, MASA-AKI TANITSU, HYE-SOOK KIM, YUSUKE WATAYA, MASAMOTO MIURA, ET AL.: "New Type of Febrifugine Analogues, Bearinga Quinolizidine Moiety, Show Potent Antimalarial Activity against P?lasmodium Malaria Parasite" J. MED. CHEM., vol. 42, 1999, pages 3163-3166, XP002337640

## Description

### Background of the invention

Malaria remains one of the most important diseases of the developing countries, killing 1-3 million people and causing disease in 300-500 million people annually. Most severe malaria is caused by the parasite *Plasmodium falciparum.*

The two most widely used antimalarial drugs, chloroquine and sulfadoxine-pyrimethamine, are failing at an accelerating rate in most malaria endemic regions with consequent increases in malaria-related morbidity and mortality. The main reasons for this failure are related to the widespread resistance of the parasite to the common antimalarials and cross-resistance to structurally unrelated drugs.

To combat malaria new drugs are desperately needed and in particular drugs that can be effective on *Plasmodium falciparum* resistant strains. Ideally the new drugs for uncomplicated Plasmodium falciparum malaria should be efficacious against drug-resistant strains, provide cure within a reasonable time (ideally three days or less) for a good compliance, be safe also for children and pregnant women, and above all be affordable at low cost (D. A. Fidock et al. Antimalarial drug discovery: efficacy models for compound screening, Nature Reviews 3, 509-520 (2004).

### Field of the invention

The present invention relates to new antimalarial compounds, in particular 4-aminoquinoline derivatives with quinolizidinyl and pyrrolizidinyl rings.

### Description of the invention

The new 4-aminoquinoline derivatives of the present invention are potent antimalarials active also on *Plasmodium falciparum* malaria strains resistant to chloroquine. The compounds of the present invention have the following general formula: 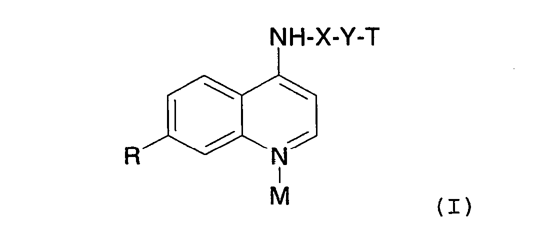 wherein
R= Cl, Br, trifluoromethyl,
M= is 0 (zero) or a complex of Au, Rh, Ru in presence of a ligand, the ligand being selected from PR'₃ wherein R' is phenyl or C₂-C₄-alkyl when M is gold; cyclooctadiene, when M is rhodium; a second identical quinoline moiety when M is ruthenium, where a group PF₆⁻ or NO₃⁻ may be present when M is gold, and a Cl⁻ may be present when M is rhodium or ruthenium;
X: is a single covalent bond,
or 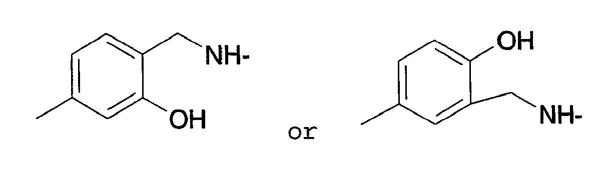 Y: is a linear or branched alkylene such as (CH₂)ₙ, where n = 0-10 and preferably 1-3;
T: 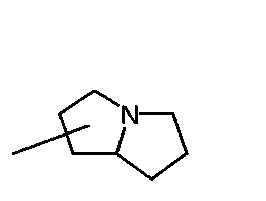 or 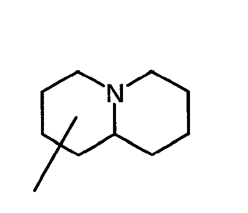 when X, defined as above, is not a single covalent bond.

Preferred compounds of the present invention are: N-(7-chloro-quinolin-4-yl)-2-(tetrahydropyrrolizin-7a-yl)-ethylamine 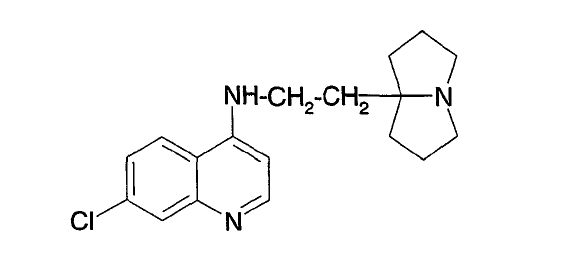 N-(7-chloro-quinolin-4-yl)-(tetrahydropyrrolizin-7a-yl-methylamine 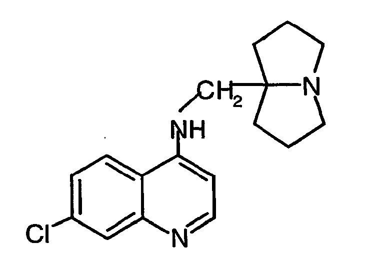 5-[(7-chloro-quinolin-4-yl)amino]-2-{[octahydroquinolizin-1-ylmethyl)-amino]-methyl}-phenol 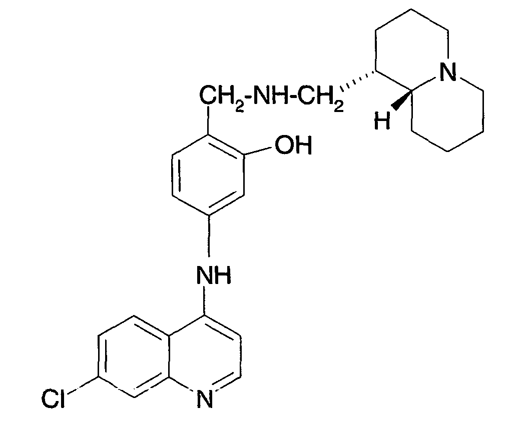 5-[(7-chloro-quinolin-4-yl)amino]-2-{[tetrahydropyrrolizin-7a-yl)-ethylamino]-methyl}-phenol 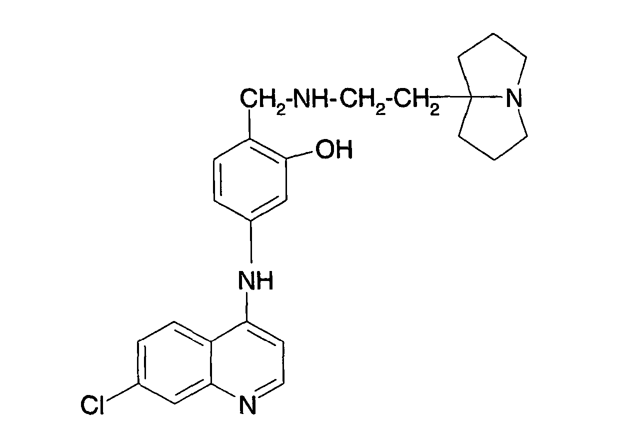 and 5-[(7-chloro-quinolin-4-yl)amino]-2-{[tetrahydropyrrolizin-7a-yl)-methylamino]-methyl}-phenol.

The compounds of the present invention were tested in vitro on chloroquine-sensitive and chloroquine-resistant strains of *Plasmodium falciparum* and IC₅₀ values ranging from 5 to 40 nM were observed.

The compounds of the invention were tested also in vivo after intraperitoneal and oral administration and the efficacy was comparable or superior to that of chloroquine. Moreover the toxicity of the compounds towards mammalian cells (murine cells WEHI clone 13) is low, with IC₅₀ >10000 nM.

Further object of the present invention is the use of the compounds according to formula (I) for all the indications that have been already described and/or suggested for chloroquine, including in a non-limitative way: prevention and/or treatment of inflammatory articular and non-articular diseases, cancer, prevention and/or treatment of other major infective diseases, including as non-limitative examples: viral infections such as avian, seasonal and pandemic influenzae, severe acute respiratory syndrome (SARS) or acquired immunodeficiency syndrome (AIDS) and bacterial infections such as tuberculosis, etc, alone or in combination with at least a proper therapeutic agents/tools.

Pharmaceutical acceptable salts, such as for example salts with inorganic and organic acids, aminoacids, are also part of the present invention. Preferred acids are hydrochloric, phosphoric, sulfuric, tartaric, citric and fumaric acid.

Depending on the specific condition or disease state to be treated, subjects may be administered compounds of the present invention at any suitable therapeutically effective and safe dosage, as may be readily determined within the skill of the art. For example, compounds of the present invention may be administered at a daily dosage between about 1 and 60 mg/kg, and more preferably between about 3 and 30 mg/kg. The compounds of the present invention can be administered in the form of any pharmaceutical formulation, the nature of which will depend upon the route of administration selected. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc. A preferred route of administration is the oral route. The following non-limitative examples further describe and enable an ordinary skilled in the art to make and use the invention.

### EXAMPLE 1. Synthesis of N-(7-chloro-quinolin-4-yl)-2-(tetrahydropyrrolizin-7a-yl)-ethylamine.

A mixture of 299 mg (1.94 mmol) of 5-(2-aminoethyl)-1-azabiciyclo[3.3.0]octane (prepared according to T. Suzuki et al., Chem. Pharm. Bull. 1997, 45, 1218), 384 mg (1.94 mmol) of 4,7-dichloroquinoline and 1.21 g (13.58 mmol)of phenol, was heated for 4 hrs at 180 °C, stirring under nitrogen atmosphere. After cooling, 2N NaOH was added to the mixture until a basic pH was reached and the product was extracted with ether. After crystallization by ether, the product had a melting point of 123.6-125.3 °C.

### EXAMPLE 2. Synthesis of N-(7-chloro-quinolin-4-yl)-(tetrahydropyrrolizin-7a-yl)-methylamine.

A mixture of 487 mg (3,46 mmol) of 5-aminomethyl-1-azabicyclo[3.3.0.]octane (prepared in according to T. Suzuki et al., Chem. Pharm. Bull. 1997, 45, 1218), 686 mg (3.46 mmol) of 4,7-dichloroquinoline and 2.28 g (24.26 mmol) of phenol, was heated for 4 hrs at 180 °C, stirring under nitrogen atmosphere. After cooling, 2N NaOH was added to the mixture until a basic pH was reached and the product was extracted with ether. After crystallization by ethyl ether/petrol ether 7/3, the product had a melting point of 109.8-111.2 °c.

### EXAMPLE 3. Synthesis of 5- [(7-chloro-quinolin-4-yl)amino]-2-{[octahydroquinolizin-1-ylmethyl)-amino-]-methyl}-phenol.

0.35 ml of aqueous formaldehyde were added to a solution of 780 mg (4.64 mmol) of aminolupinane (prepared from lupinine according to F. Sparatore et al. *Farmaco*, *Ed. Sci.* 1969, 24, 587) and 720 mg (4.64 mmol) of 3-acetamidophenol in 3.5 ml of ethanol. The mixture was heated under reflux for 24 hours, stirring under nitrogen. After cooling, the solvent was removed under reduced pressure and the crude material was purified by flash chromatography on silica gel column using dichloromethane/methanol 92/8 as eluent. The obtained product, N-(3-hydroxy-4-{[octahydroquinolizin-1-yl-methyl)-amino]-methyl}-phenyl)-acetamide, was washed with ethyl ether.

N-(3-hydroxy-4-{[octahydroquinolizin-1-yl-methyl)-amino]-methyl}-phenyl)-acetamide (380 mg, 0.93 mmol) was dissolved in 3 ml of 20% HCl and the solution was heated under reflux, under nitrogen for 8 hours. After evaporation under reduced pressure, the residue was dissolved in 3 ml of ethanol and 4,7-dichloroquinoline (184 mg, 0.93 mmol) was added to the solution, and the mixture was heated under reflux for 16 hours. After evaporation of the solvent, the residue was purified by flash chromatography on silica gel column eluting with dichloromethane/methanol gradient. To liberate the free base compound, the eluted product was dissolved in water and the solution basified by addition of saturated NaHCO₃ (added until no more precipitate was formed) and then extracted with dichloromethane. The organic phase was dried on anhydrous sodium sulphate and evaporated. The residue, washed with ether, had a melting point 134-135 °C.

### EXAMPLE 4. 5-[(7-chloro-quinolin-4-yl)amino]-2-{[tetrahydropyrrolizin-7a-yl)-ethylamino]-methyl}-phenol.

Aqueous formaldehyde (0.32 ml) was added to a solution of 5-(2-aminoethyl)-1-azabicyclo[3.3.0]octane (752 mg, 4.87 mmol) and 3-acetamidophenol (736 mg, 4.87 mmol) in 3.7 ml of ethanol. The mixture was heated under reflux for 24 hours, stirring under nitrogen. After cooling, the solvent was removed under reduced pressure and the crude material was purified by flash chromatography on silica gel column using dichloromethane/methanol/ conc. NH₃, (10/3/0.1) as eluent. The obtained product, N-(3-hydroxy-4-{[2-(tetrahydropyrrolizin-7a-yl)-ethyl-amino]-methyl}-phenyl)-acetamide, washed with a mixture of ether and dichloromethane 1/1, was used as such for the following reaction.
N-(3-hydroxy-4-{[2-(tetrahydro-pyrrolizine-7a-yl)-ethylamino]-methyl}-phenyl)-acetamide (175 mg, 0.55 mmol) was dissolved in 3 ml of 20% HCl and the solution was heated under reflux, under nitrogen for 8 hours. After evaporation under reduced pressure, the residue was dissolved in 3 ml of ethanol and 4,7-dichloroquinoline (109 mg, 0.55 mmol) was added to the solution, and the mixture was heated under reflux for 18 hours. After evaporation of the solvent the residue was purified by flash chromatography on silica gel column eluting with dichloromethane/methanol gradient. To liberate the free base compound, the eluted product was dissolved in water and the solution basified by addition of NH₃ until pH 8-9 and then extracted with dichloromethane. The organic phase was dried on anhydrous sodium sulphate and evaporated. The residue, washed with ether, was dissolved in abs. EtOH and added with an excess (3 ml) of a 1 M HCl ethanolic solution. After evaporation of the solvent, the residue was washed with a mixture of ether/EtOH 7.5:2.5 and had m.p. 247-251°C.

### EXAMPLE 5. 5-[(7-chloro-quinolin-4-yl)amino]-2-{[tetrahydropyrrolizin-7a-yl)-methylamino]-methyl}-phenol.

Aqueous formaldehyde (0.32 ml) was added to a solution of 5-(2-aminomethyl)-1-azabicyclo[3.3.0]octane (4.87 mmol) and 3-acetamidophenol (4.87 mmol) in 3.7 ml of ethanol. The mixture is heated under reflux for 24 hours, stirring under nitrogen. After cooling the solvent was removed under reduced pressure and the crude material was purified by silica gel flash column chromatography using dichloromethane/methanol/ conc. NH₃, (10/3/0.1) as eluent. The obtained product, N-(3-hydroxy-4-{[2-(tetrahydro-pyrrolizin-7a-yl)-methylamino]-methyl}-phenyl)-acetamide, washed with a mixture of ether and dichloromethane 1/1, was used as such for the following reaction.
N-(3-hydroxy-4-{[2-(tetrahydro-pyrrolizine-7a-yl)-methylamino]-methyl}-phenyl)-acetamide (0.55 mmol) is dissolved in 3 ml of 20% HCl and the solution was heated under reflux, under nitrogen for 8 hours. After evaporation under reduced pressure, the residue was dissolved in 3 ml of ethanol and 4,7-dichloroquinoline (109 mg, 0.55 mmol) was added to the solution, and the mixture was heated under reflux for 8 hours. After evaporation of the solvent the residue was dissolved in water and the solution basified by addition of NH₃ until pH 8-9 and then extracted with dichloromethane. The organic phase was dried on anhydrous sodium sulphate and evaporated. The residue, after purification by flash column chromatography on silica gel eluting with dichloromethane/methanol gradient, had m.p. 109-120°C.

### Example 6. Gold complex of compound of example 2

200 mg (0.4 mmol) of triphenylphosphine gold chloride dissolved under reflux in 20 ml of acetonitrile were added with 148.8 mg (0.8 mmol) of potassium hexafluorophosphate (KPF₆), heating for 30 minutes. 247.5 mg (0.82 mmol) of N-(7-chloroquinolin-4-yl)-(tetrahydropyrrolizin-7a-yl)methylamine were added, the mixture was refluxed under nitrogen for 48 hours and after cooling the resulting precipitate was filtered. The filtrate was concentrated, added with few drops of ethyl ether and stored in the refrigerator. The separated solid was filtered and washed with anhydrous ether/acetonitrile (1:3). The solution was concentrated again, treated with ether and stored in the refrigerator. This procedure was repeated several times, each one filtering off the precipitate. Finally the solution was evaporated to dryness and the residue iwas washed with ether and dried.

### EXAMPLE 7. Biological data

The compounds of the present invention have been tested in vitro and found to be potent on chloroquine-sensitive (CQ-S) *Plasmodium falciparum* strain D10 and on chloroquine-resistant (CQ-R) *Plasmodium falciparum* strain W2. The results are reported in the following table.

| Compounds | D10 (CQ-S) (IC₅₀ ng/ml) | W2 (CQ-R) (IC₅₀ ng/ml) |
|---|---|---|
| Example 1 | 1.7 ± 1.4 | 17.3 ± 2.4 |
| Example 2 | 9.2 ± 2.2 | 8.7 ± 3.5 |
| Example 3 | 10.3 ± 1.9 | 11.4 ± 2.1 |
| Example 4 | 17.2 ± 1.9 | < 80 |
| Chloroquine | 9.7 ± 3.6 | 170.1 ± 41.3 |

| | | |
|---|---|---|
| n=4 ± SD | | |

## Claims

1. Compounds of formula (I) 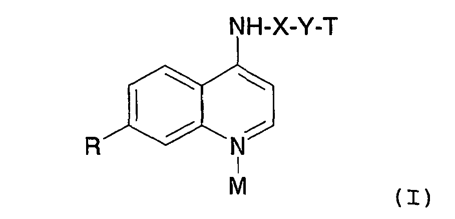 wherein
R= Cl, Br, trifluoromethyl;
M= is 0 (zero) or a complex of Au, Rh, Ru in presence of a ligand, the ligand being selected from PR'₃ wherein R' is phenyl or C₂-C₄-alkyl when M is gold; cyclooctadiene, when M is rhodium; a second identical quinoline moiety when M is ruthenium, wherein a group PF₆⁻ or NO₃⁻ may be present when M is gold and a Cl⁻ may be present when M is rhodium or ruthenium;
X: is a single covalent bond,
or 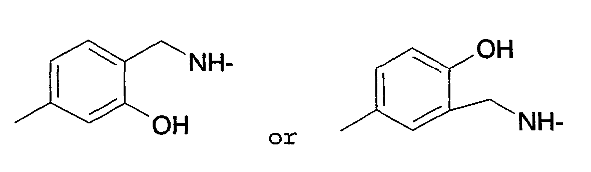 Y: is a linear or branched alkylene such as (CH₂)n, where n = 0-10;
T: 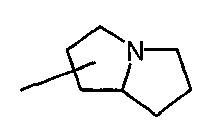 or 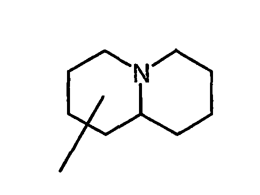 when X, defined as above, is not a single covalent bond and salts thereof.

2. A compound of formula (I) according to claim 1, wherein n is 1-3.

3. A compound according to claim 1, that is N-(7-chloroquinolin-4-yl)-2-(tetrahydropyrrolizin-7a-yl)-ethylamine.

4. A compound according to claim 1, that is N-(7-chloroquinolin-4-yl)-(tetrahydropyrrolizin-7a-yl)-methylamine.

5. A compound according to claim 1, that is 5-[(7-chloroquinolin-4-yl)amino]-2-{[octahydroquinolizin-1-ylmethyl)-amino]-methyl}-phenol.

6. A compound according to claim 1, that is 5-[(7-chloroquinolin-4-yl)amino]-2-{[tetrahydropyrrolizin-7a-yl)-ethylamino]-methyl}-phenol.

7. A compound according to claim 1, that is 5-[(7-chloroquinolin-4-yl)amino]-2-{[tetrahydropyrrolizin-7a-yl)-methylamino]-methyl}-phenol.

8. Pharmaceutical compositions containing a compound of formula (I) according to claim 1 as active ingredient and pharmaceutically acceptable adjuvants or carriers.

9. Use of a compound according to one of the claims 1-7 for the manufacture of a medicament for prevention and/or treatment of malaria.

10. Use of a compound according to one of the claims 1-7 for the manufacture of a medicament for prevention and/or treatment of chloroquine-resistant *Plasmodium falciparum* malaria strains.

11. Use of a compound according to one of the claims 1-7 for the manufacture of a medicament for prevention and/or treatment of inflammatory articular and non-articular diseases.

12. Use of a compound according to one of the claims 1-7 for the manufacture of a medicament for prevention and/or treatment of cancer.

13. Use of a compound according to one of the claims 1-7 for the manufacture of a medicament for prevention and/or treatment of infective diseases.

14. Use of a compound according to one of the claims 1-7 for the manufacture of a medicament for prevention and/or treatment of viral infections.

15. Use of a compound according to one of the claims 1-7 for the manufacture of a medicament for prevention and/or treatment of avian, seasonal and pandemic influenzae.

16. Use of a compound according to one of the claims 1-7 for the manufacture of a medicament for prevention and/or treatment of severe acute respiratory syndrome (SARS).

17. Use of a compound according to one of the claims 1-7 for the manufacture of a medicament for prevention and/or treatment of acquired immunodeficiency syndrome (AIDS).

18. Use of a compound according to one of the claims 1-7 for the manufacture of a medicament for prevention and/or treatment of bacterial infections.

19. Use of a compound according to one of the claims 1-7 for the manufacture of a medicament for prevention and/or treatment of tuberculosis.

20. Pharmaceutical compositions comprising at least one compound as claimed in one of the claims 1-7 as an active ingredient.

## Patentansprüche

1. Verbindungen der Formel (1) 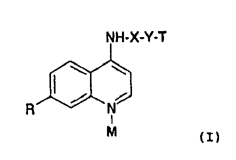 wobei
R= Cl, Br, Trifluormethyl;
M = 0 (null) oder ein Komplex aus Au, Rh, Ru bei Vorhandensein eines Liganden ist, wobei der Ligand ausgewählt wird aus PR'₃, wobei R' Phenyl oder C₂-C₄-Alkyl ist, wenn M Gold ist; Cyclooctadien, wenn M Rhodium ist; eine zweite identische Chinolin-Hälfte, wenn M Ruthenium ist, wobei eine Gruppe PFC oder NO₃ vorhanden sein kann, wenn M Gold ist und ein Cl vorhanden sein kann, wenn M Rhodium oder Ruthenium ist;
X: eine einzelne kovalente Bindung ist,
oder 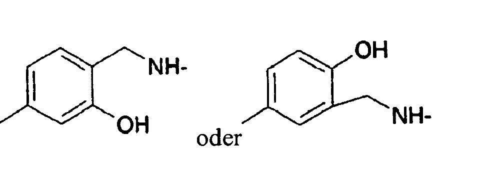 Y: ein lineares oder verzweigtes Alken wie z.B. (CH₂)n ist, wobei n = 0-10;
T: 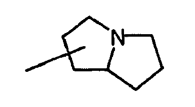 oder 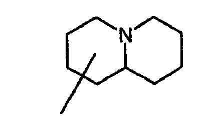 wenn X, definiert wie vorstehend, keine einzelne kovalente Bindung oder Salz davon ist.

2. Verbindung der Formel (1) nach Anspruch 1, wobei n 1-3 ist.

3. Verbindung nach Anspruch 1, die N-(7-Chlorchinolin-4-yl)-2-(Tetrahydropyrrolizin-7a-yl)-Ethylamin ist.

4. Verbindung nach Anspruch 1, die N-(7-Chlorchinolin-4-yl)-(Tetrahydropyrrolizin-7a-yl)-Methylamin ist.

5. Verbindung nach Anspruch 1, die 5-[(7-Chlorchinolin-4-yl)amino]-2-f[(Oktahydropyrrolizin-1-ylmethyl)-amino]-Methyl)-Phenol ist.

6. Verbindung nach Anspruch 1, die 5-[(7-Chlorchinolin-4-yl)amino]-2-{(Tetrahydropyrrolizin-7a-yl)-Ethylamin]-Methyl)-Phenol ist.

7. Verbindung nach Anspruch 1, die 5-[(7-Chlorchinolin-4-yl)amino]-2-{[Tetrahydropyrrolizin-7a-yl)-Methylamin]-Methyl}-Phenol ist.

8. Pharmazeutische Verbindungen, die eine Verbindung der Formel (1) nach Anspruch 1 als Wirkstoff und pharmazeutisch akzeptable Hilfsstoffe oder Träger enthalten.

9. Verwendung einer Verbindung nach einem der Ansprüche 1-7 zur Herstellung eines Medikaments zur Vorbeugung und/oder Behandlung von Malaria.

10. Verwendung einer Verbindung nach einem der Ansprüche 1-7 zur Herstellung eines Medikaments zur Vorbeugung und/oder Behandlung von chloroquinresistenten *Plasmodium falciparum* Malariaerregern.

11. Verwendung einer Verbindung nach einem der Ansprüche 1-7 zur Herstellung eines Medikaments zur Vorbeugung und/oder Behandlung von entzündlichen Gelenk- und Nichtgelenkerkrankungen.

12. Verwendung einer Verbindung nach einem der Ansprüche 1-7 zur Herstellung eines Medikaments zur Vorbeugung und/oder Behandlung von Krebs.

13. Verwendung einer Verbindung nach einem der Ansprüche 1-7 zur Herstellung eines Medikaments zur Vorbeugung und/oder Behandlung von infektiösen Krankheiten.

14. Verwendung einer Verbindung nach einem der Ansprüche 1-7 zur Herstellung eines Medikaments zur Vorbeugung und/oder Behandlung von Virusinfektionen.

15. Verwendung einer Verbindung nach einem der Ansprüche 1-7 zur Herstellung eines Medikaments zur Vorbeugung und/oder Behandlung von Vogelgrippe, saisonaler Grippe und Pandemiegrippe.

16. Verwendung einer Verbindung nach einem der Ansprüche 1-7 zur Herstellung eines Medikaments zur Vorbeugung und/oder Behandlung des schweren akuten Atemwegssyndrom (SARS).

17. Verwendung einer Verbindung nach einem der Ansprüche 1-7 zur Herstellung eines Medikaments zur Vorbeugung und/oder Behandlung des erworbenen Immundefektsyndroms (AIDS).

18. Verwendung einer Verbindung nach einem der Ansprüche 1-7 zur Herstellung eines Medikaments zur Vorbeugung und/oder Behandlung von bakteriellen Infektionen.

19. Verwendung einer Verbindung nach einem der Ansprüche 1-7 zur Herstellung eines Medikaments zur Vorbeugung und/oder Behandlung von Tuberkulose.

20. Pharmazeutische Verbindungen, die mindestens eine Verbindung nach einem der Ansprüche 1-7 als Wirkstoff enthalten.

## Revendications

1. Composés de formule (1) 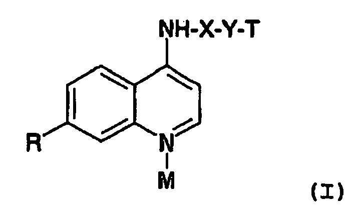 dans laquelle
R = Cl, Br, trifluorométhyle ;
M = vaut 0 (zéro) ou un complexe de Au, Rh, Ru en présence d'un ligand, le ligand étant choisi parmi PR'₃ où R' est un phényle ou un alkyle en C₂ à C₄ quand M est l'or ; le cyclooctadiène, quand M est le rhodium ; une deuxième fraction de quinoline identique quand M est le ruthénium, où un groupe PF₆⁻ ou NO₃⁻ peut être présent quand M est l'or et un Cl⁻ peut être présent quand M est le rhodium ou le ruthénium ;
X : est une simple liaison covalente,
ou 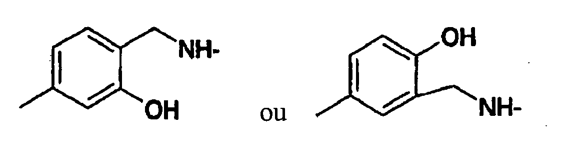 Y : est un alkylène linéaire ou ramifié tel que (CH₂) n, où n = 0 à 10
T: 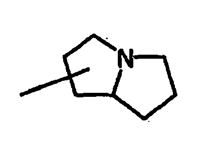 ou 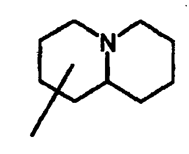 quand X, défini comme ci-dessus, n'est pas une simple liaison covalente, et leurs sels.

2. Composé de formule (I) selon la revendication 1, dans lequel n vaut 1 à 3.

3. Composé selon la revendication 1, qui est la N-(7-chloroquinolin-4-yl)-2-(tétrahydropyrrolizin-7a-yl)-éthylamine.

4. Composé selon la revendication 1, qui est la N-(7-chloroquinolin-4-yl)-2-(tétrahydropyrrolizin-7a-yl)-méthylamine.

5. Composé selon la revendication 1, qui est le 5-[(7-chloroquinolin-4-yl)-amino]-2- {[octahydroquinolizin-1-ylméthyl)-amino]-méthyl}-phénol.

6. Composé selon la revendication 1, qui est le 5-[(7-chloroquinolin-4-yl)amino]-2-{[tétrahydropyrrolizin-7a-yl)-éthylamino]-méthyl}-phénol.

7. Composé selon la revendication 1, qui est le 5-[(7-chloroquinolin-4-yl)amino]-2-{[tétrahydropyrrolizin-7a-yl)-méthylamino]-méthyl}-phénol.

8. Compositions pharmaceutiques contenant un composé de formule (I) selon la revendication 1 comme ingrédient actif et adjuvants ou véhicules pharmaceutiquement acceptables.

9. Utilisation d'un composé selon une des revendications 1 à 7 pour la fabrication d'un médicament destiné à la prévention et/ou au traitement de la malaria.

10. Utilisation d'un composé selon une des revendications 1 à 7 pour la fabrication d'un médicament destiné à la prévention et/ou au traitement des souches de malaria *Plasmodium falciparum* résistantes à la chloroquine.

11. Utilisation d'un composé selon une des revendications 1 à 7 pour la fabrication d'un médicament destiné à la prévention et/ou au traitement des maladies inflammatoires articulaires et non articulaires.

12. Utilisation d'un composé selon une des revendications 1 à 7 pour la fabrication d'un médicament destiné à la prévention et/ou au traitement du cancer.

13. Utilisation d'un composé selon une des revendications 1 à 7 pour la fabrication d'un médicament destiné à la prévention et/ou au traitement des maladies infectieuses.

14. Utilisation d'un composé selon une des revendications 1 à 7 pour la fabrication d'un médicament destiné à la prévention et/ou au traitement des infections virales.

15. Utilisation d'un composé selon une des revendications 1 à 7 pour la fabrication d'un médicament destiné à la prévention et/ou au traitement de l'influenza aviaire saisonnière et pandémique.

16. Utilisation d'un composé selon une des revendications 1 à 7 pour la fabrication d'un médicament destiné à la prévention et/ou au traitement du syndrome respiratoire aigu grave (SARS).

17. Utilisation d'un composé selon une des revendications 1 à 7 pour la fabrication d'un médicament destiné à la prévention et/ou au traitement du syndrome de l'immunodéficience acquise (SIDA).

18. Utilisation d'un composé selon une des revendications 1 à 7 pour la fabrication d'un médicament destiné à la prévention et/ou au traitement des infections bactériennes.

19. Utilisation d'un composé selon une des revendications 1 à 7 pour la fabrication d'un médicament destiné à la prévention et/ou au traitement de la tuberculose.

20. Compositions pharmaceutiques comprenant au moins un composé selon l'une de revendications 1 à 7 comme ingrédient actif.
